# EUROPÄISCHE PATENTSCHRIFT

(11) **EP 1 165 497 B1**
(45) Veröffentlichungstag und Bekanntmachung des Hinweises auf die Patenterteilung: **25.02.2004**
(21) Anmeldenummer: 00912618.6
(22) Anmeldetag: 17.03.2000
(51) Int. Cl.: C07C 253/30, C07C 255/25, C07D 295/14

(54) **VERFAHREN ZUR HERSTELLUNG VON KÖRNIGEN N-ALKYLAMMONIUMACETONITRIL-SALZEN**
METHOD OF PREPARING GRANULAR N-ALKYL-AMMONIUM ACETONITRILE SALTS
PROCEDE DE FABRICATION DE SELS DE N-ALKYLAMMONIUMACETONITRILE EN GRAINS

(30) Priorität: 29.03.1999 DE 19913995
(43) Veröffentlichungstag der Anmeldung: 02.01.2002
(73) Patentinhaber: BASF AKTIENGESELLSCHAFT, 67056 Ludwigshafen (DE)
(72) Erfinder: SCHÖNHERR, Michael, D-67227 Frankenthal (DE); KINDER, Hans-Jürgen, D-67346 Speyer (DE); MUNDINGER, Klaus, D-67117 Limburgerhof (DE); SCHÜRMANN, Gregor, D-68723 Schwetzingen (DE)
(86) Internationale Anmeldenummer: PCT/EP2000/002378
(87) Internationale Veröffentlichungsnummer: WO 2000/058273

(56) Entgegenhaltungen:
- EP-A- 0 149 264
- WO-A-98/23531
- DE-A- 19 740 669
- DE-A- 19 740 671
- US-A- 4 213 924
- US-A- 5 354 493

## Beschreibung

Die vorliegende Erfindung betrifft ein verbessertes Verfahren zur Herstellung von körnigen N-Alkylammoniumacetonitril-Salzen der allgemeinen Formel I

R²R³N⁺R¹-CR⁴R⁵-CN Y⁻ (I)

in der
- R¹: eine C₁- bis C₂₄-Alkylgruppe, welche durch nicht benachbarte Sauerstoffatome unterbrochen sein oder zusätzlich Hydroxylgruppen tragen kann, eine C₄- bis C₂₄-Cycloalkylgruppe, eine C₇- bis C₂₄-Alkarylgruppe oder eine Gruppierung der Formel -CR⁴R⁵-CN bedeutet,
- R² und R³: jeweils unabhängig voneinander die Bedeutung von R¹ aufweisen oder zusammen einen gesättigten vier- bis neungliedrigen Ring mit wenigstens einem C-Atom und wenigstens einem weiteren Heteroatom aus der Gruppe Sauerstoff, Schwefel und Stickstoff-darstellen,
- R⁴ und R⁵: jeweils unabhängig voneinander Wasserstoff, C₁- bis C₂₄-Alkylgruppen, welche durch nicht benachbarte Sauerstoffatome unterbrochen sein oder zusätzlich Hydroxylgruppen tragen können, C₄- bis C₂₄-Cycloalkylgruppen oder C₇- bis C₂₄-Alkarylgruppen bezeichnen und
- Y⁻: für ein Sulfat- oder Hydrogensulfat-Anion in der entsprechenden stöchiometrischen Menge steht,
aus einer wäßrigen Lösung der Verbindung der allgemeinen Formel II

R²R³N⁺R¹-CR⁴R⁵-CN R⁶O-SO₂-O⁻ (II)

gemäß Anspruch 1 in der R¹ bis R⁵ die oben genannten Bedeutungen aufweisen und R⁶ für C₁- bis C₄-Alkyl steht.

N-Alkylammoniumacetonitril-Salze wie N-Methylmorpholiniumacetonitrilsulfat und -hydrogensulfat werden insbesondere als Aktivatoren in Form von Feststoffen für die Niedertemperaturbleiche in Waschmitteln benötigt. In der WO 98/23531 werden Granulate solcher verbindungen, welche zusätzlich Trägermaterialien enthalten können, für diese Anwendung beschrieben. Für die Herstellung derartiger Sulfat- oder Hydrogensulfat-Granulate wird dort empfohlen, von den Methylsulfat-Salzen auszugehen.

Bekannte Verfahren zur Herstellung dieser festen N-Alkylammoniumacetonitril-sulfate bzw. -hydrogensulfate sind jedoch noch verbesserungsbedürftig. Daher war es die Aufgabe der vorliegenden Erfindung, ein derartiges Verfahren bereitzustellen, welches die Nachteile des Standes der Technik vermeidet.

Demgemäß wurde das vorliegende Verfahren gefunden, welches dadurch gekennzeichnet ist, daß man die wäßrige Lösung der Verbindung II bei einer Temperatur von 80°C bis 250°C und einem Druck von 100 mbar bis 2 bar zu einer Schmelze eindampft, anschließend die Schmelze erstarren läßt, wobei während oder im Anschluß an das Eindampfen übliche Trägermaterialien und/oder Hilfsmittel zugegeben werden können, und die erhaltene erstarrte Verbindung I in die gewünschte körnige Form überführt, dadurch gekennzeichnet, daß die Hydrolyse des Gegenions R⁶-O-SO₂-O⁻ zu Y⁻ thermisch, d.h. ohne Zugabe von Säure, erfolgt.

Beim Eindampfschritt wird der wäßrigen Lösung der Verbindung II Wasser und gleichzeitig der entsprechende C₁- bis C₄-Alkohol entzogen, welcher bei der teilweisen oder vollständigen thermischen Hydrolyse des Gegenions Y frei wird.

Der Rest R¹, der in der Regel durch die Alkylierung des N-Atoms entstanden ist, bedeutet beispielsweise:
- einen geradkettigen oder verzweigten längeren oder insbesondere kürzeren Alkylrest mit 1 bis 24 C-Atomen, wobei auch ungesättigte Reste, insbesondere ungesättigte Fettsäurereste, geeignet sind, z.B. Methyl, Ethyl, Propyl, iso-Propyl, Butyl, iso-Butyl, sec.-Butyl, tert.-Butyl, Pentyl, Hexyl, Heptyl, Octyl, 2-Ethylhexyl, Nonyl, iso-Nonyl, Decyl, Undecyl, Dodecyl, Tridecyl, iso-Tridecyl, Myristyl, Cetyl, Stearyl oder Oleyl;
- einen Alkoxyalkylrest, z.B. Methoxymethyl, 2-Methoxyethyl, 3-Methoxypropyl, 4-Methoxybutyl, 2-Ethoxyethyl oder 3-Ethoxypropyl;
- einen Hydroxyalkylrest, z.B. Hydroxymethyl, 2-Hydroxyethyl, 2-Hydroxypropyl, 3-Hydroxy-2-butyl oder 4-Hydroxybutyl;
- einen aus wiederkehrenden C₂- bis C₄-Alkylenoxid-Einheiten wie Ethylenoxid, Propylenoxid oder Butylenoxid aufgebauten Rest, der durch eine Hydroxylgruppe oder eine Alkoxygruppe terminiert sein kann, z.B.-(C₂H₄O)ₙ-H oder - (C₂H₄O)ₙ-R⁷, - (C₃H₆O)ₘ-H oder - (C₃H₆O)ₘ-R⁷, - (C₄H₈O)ₖ-H oder - (C₄H₈O)ₖ-R⁷ (n = 2 bis 11, m = 2 bis 7, k = 2 bis 5, R⁷ = Methyl oder Ethyl);
- eine Cycloalkylgruppe wie Cyclopentyl, Cyclohexyl oder Cycloheptyl;
- eine Aralkylgruppe wie Benyl, 2-Phenylethyl, 3-Phenylpropyl oder 4-Phenylbutyl;
- eine Gruppierung der Formel -CH₂-CN, -CH(CH₃)-CN oder - C(CH₃)₂CN.

Bevorzugte Bedeutungen für R¹ sind eine C₁- bis C₄-Alkylgruppe oder ein Benzylrest.

Die Bedeutungen für die Reste R⁴ und R⁵ sind prinzipiell die gleichen wie für R¹ (mit Ausnahme von -CR⁴R⁵-CN) , zusätzlich bezeichnen sie auch Wasserstoff. Bevorzugte Bedeutungen für R⁴ und R⁵ sind Wasserstoff, Methyl und Ethyl, insbesondere stehen beide für Wasserstoff.

In einer bevorzugten Ausführungsform bedeutet R¹ eine C₁- bis C₄-Alkylgruppe oder einen Benzylrest und R⁴ und R⁵ bezeichnen beide gleichzeitig Wasserstoff.

Die Bedeutungen für die Reste R² und R³ sind für offenkettige alicyclische Strukturen prinzipiell die gleichen wie für R¹. Zusätzlich können R² und R³ zusammen mit dem Ammonium-N-Atom einen gesättigten heterocyclischen Ring darstellen. Dabei kommen insbesondere solche in Betracht, die neben dem Ammonium-N-Atom kein, ein oder zwei weitere Heteroatome aus der Gruppe Sauerstoff, Schwefel und Stickstoff, vor allem aus der Gruppe Sauerstoff und Stickstoff, enthalten. Bevorzugte Ringgrößen sind fünf-, sechsund siebengliedrige Ringe. Beispiele für hierbei in Betracht kommende heterocyclische Systeme sind Imidazolidin, 1,2,3-Triazolidin und Piperazin.

Besonders bevorzugt werden Systeme, bei denen R² und R³ zusammen einen gesättigten sechsgliedrigen Ring mit 5 C-Atomen oder mit 4 C-Atomen und einem Sauerstoff- oder einem Stickstoffatom darstellen. Hierbei handelt es sich insbesondere um Piperidin- und Morpholin-Systeme.

Der Rest R⁶ steht beispielsweise für Methyl, Ethyl, Propyl, iso-Propyl, Butyl, iso-Butyl, sec.-Butyl oder tert.-Butyl, vorzugsweise für Methyl.

Ganz besonders bevorzugt wird nach dem erfindungsgemäßen Verfahren körniges N-Methylmorpholiniumacetonitril-hydrogensulfat aus einer wäßrigen Lösung von N-Methylmorpholiniumacetonitril-methylsulfat hergestellt.

Die Eindampfung wird - in Abhängigkeit vom Druck - bei Temperaturen von 80°C bis 250°C, vorzugsweise von 90°C bis 200°C, insbesondere bei 100°C bis 160°C, durchgeführt. Im bevorzugten Druckbereich sind Temperaturen von 100°C bis 160°C besonders günstig, weil dann eine hinreichende Abreicherung an Wasser und Alkohol stattfindet und die Zersetzung des Acetonitrils I zur entsprechenden Amid-Stufe noch vernachlässigbar gering ist.

Die Eindampfung wird bei einem Druck von 10 mbar bis 2 bar, vorzugsweise von 100 mbar bis 1,1 bar, insbesondere bei 250 bis 900 mbar, durchgeführt. Bevorzugt wird die Eindampfung im leichten Vakuum, weil sich unter diesen Bedingungen flüchtige Nebenkomponenten leichter austreiben lassen.

Der Eindampfungsschritt wird vorzugsweise in einer kontinuierlichen Fahrweise durchgeführt, beispielsweise in einem Sambay-Verdampfer, einem Dünnschichtkontakttrockner, einem Fallfilmverdampfer oder einem Rohrbündelwärmetauscher. Die Eindampfung kann jedoch auch diskontinuierlich, d.h. in einem Batch-Prozeß, z.B. in einem Kessel, erfolgen.

Die erforderliche Verweilzeit für den Eindampfuhgsschritt ist abhängig von der gewählten Temperatur und dem gewählten Druck und liegt in der Regel im Bereich von wenigen Minuten bis zu mehreren Stunden. Ein tpypischer derartiger Bereich ist von 2 Minuten bis 15 Stunden, insbesondere von 5 Minuten bis 5 Stunden. Im bevorzugten Temperatur- und Druckbereich ist eine Verweilzeit von 5 bis 15 Minuten besonders günstig. Bei zu langen Verweilzeiten kann die Zersetzung des Produktes einsetzen. Die Verweilzeitverteilung sollte eng sein, im Fall einer kontinuierlichen Fahrweise sollte die Plugflow-Strömungsform angestrebt werden, um die Bildung von Neben- und Zersetzungsprodukten zu vermeiden.

In einer besonders bevorzugten Ausführungsform wir die Eindampfung kontinuierlich mit einer Verweilzeit von 5 bis 15 Minuten durchgeführt.

Aus dem Eindampfungsschritt treten die Produktschmelze und dampfförmige Brüden in Form von überwiegend Wasser- und Alkoholdampf aus. Im erfindungsgemäßen Verfahren kann eine deutliche Reduzierung des Energieeinsatzes erreicht werden, indem die Kondensation der bei der Eindampfung entstehenden Brüden zur Vorwärmung der wäßrigen Lösung der Verbindung II benutzt wird. Apparatetechnisch können dafür beispielsweise Rohrbündel- oder Plattenwärmetauscher eingesetzt werden.

Die so erzeugte Schmelze hat üblicherweise einen Wassergehalt von maximal 30 Gew.-%, insbesondere von maximal 20 Gew.-%, vor allem von 1 bis 10 Gew.-%. Der Anteil des Sulfat- bzw. Hydrogensulfat-Salzes I in der Schmelze beträgt in der Regel mindestens 50 Gew.-%, insbesondere mindestens 70 Gew.-%, vor allem mindestens 80 Gew.-%. Die als Ausgangsmaterial eingesetzte wäßrige Lösung der Verbindung II hat üblicherweise einen Feststoffgehalt von 5 bis 80 Gew.-%, insbesondere von 10 bis 75 Gew.-%, vor allem von 25 bis 65 Gew.-%.

Vor dem Erstarren kann die Schmelze mit üblichen Trägermaterialien und/oder Hilfsmitteln vermischt werden. Diese Trägermaterialien können je nach Anwendungsgebiet wasserlöslich oder wasserunlöslich sein. Beispiele für solche Trägermaterialien, die man während oder im Anschluß an das Eindampfen zugibt, sind Natriumsulfat, Kieselsäuren und Zeolithe. Mann kann auch mehrere der genannten Trägermaterialien, d.h. Gemische hieraus, zugeben. Neben Trägermaterialien können der Schmelze auch funktionelle Hilfsstoffe wie Tenside zugesetzt werden.

Das Vermischen mit den Trägermaterialien bzw. Hilfsstoffen kann entweder in einem gesonderten Apparat, z.B. in einem Rührbehälter oder in einem kontinuierlichen Mischer, erfolgen. Dann kann die Mischung anschließend durch Kontaktkühlung, z.B. auf einer Kühlwalze, auf einem Kühlband oder in einem Kühlmischer, oder durch konvektive Kühlung, z.B. in einem Prillturm oder in einer Sprühgranulationsanlage, erstarrt werden. Es ist jedoch auch möglich, das Mischen und Erstarren der Schmelze im selben Apparat, z.B. in einem Extruder, Knetreaktor oder Kühlmischer, durchzuführen.

Der Kühlprozeß kann bei Überdruck, Normaldruck oder im Vakuum durchgeführt werden. Hier kann im Prinzip innerhalb der gleichen Druckbereichen wie beim Eindampfungsschritt gearbeitet werden. Bevorzugt wird beim Kühlprozeß eine Verfah-rensführung unter Normaldruck oder - wie beim Eindampfungsschritt - unter leichtem Vakuum infolge einer Absaugung.

Die Kühltemperaturen können im Bereich von der Erstarrungstemperatur der Schmelze bis zu stark negativen Temperaturen, wie sie beispielsweise bei der Kühlung mit flüssigem Stickstoff (-196°C) auftreten, liegen. Bevorzugt werden Kühltemperaturen im Bereich von -50°C bis +30°C, insbesondere von -20°C bis +20°C.

Die erforderliche Verweilzeit des Erstarrungsprozesses hängt von den Kristallisationseigenschaften des Stoffgemisches ab und liegt in der Regel im Bereich von wenigen Minuten (z.B. 5 Minuten) bis zu einer Stunde. Eine innige Durchmischung, wie sie beispielsweise in Extrudern oder Knetreaktoren auftritt, kann die erforderliche Kristallisationszeit in vielen Fällen erheblich verkürzen.

Nach dem Erstarrungsprozeß liegt das Produkt, welches das Salz I neben eventuell noch vorhandenem Ausgangsmaterial II und gegebenenfalls Trägermaterialien und/oder Hilfsmittel enthält, in der Regel als Feststoff mit einer breiten Partikelgrößenverteilung vor und genügt so beispielsweise den Anforderungen an ein Waschmittelgranulat noch nicht. Die gewünschte körnige Form hinsichtlich der Partikelgröße kann durch geeignete Sieb- und/oder Mahlschritte mit üblichen verfahrenstechnischen Einrichtung herbeigeführt werden. Übliche Partikelgrößen liegen bei 100 bis 5000 Mikrometer, insbesondere bei 300 bis 2000 Mikrometer.

Das erfindungsgemäße Verfahren weist eine Reihe von Vorteilen auf. So ermöglicht es eine Umwandlung mit niedrigem Nebenproduktspiegel und die Reduzierung von flüchtigen Nebenkomponenten. Aus der bevorzugten kontinuierlichen Fahrweise resultieren hohe Ausbeuten. Durch den geringen Hold-Up in der Anlage ist die kontinuierliche Fahrweise auch sicherheitstechnisch vorteilhaft. Durch die Möglichkeit der Wärmerückgewinnung ergibt sich eine energetisch günstige Prozeßführung. Es können die verschiedensten Trägermaterialien und Hilfsmittel im Granulationsprozeß mitverwendet werden. Außerdem ist die Steuerung der Partikelgrößenverteilung im Granulationsprozeß gut durchführbar.

### Beispiel

In einem 80 Liter-Glasbehälter wurden 60 Liter einer 65 gew.-%igen wäßrigen Lösung von N-Methylmorpholiniumacetonitril-methylsulfat vorgelegt. Die Lösung wurde auf 110°C erhitzt und bei einem Druck von 600 mbar über einen Zeitraum von 3 Stunden eingedampft. Die Schmelze, welche einen Gehalt an N-Methylmorpholiniumacetonitrilhydrogensulfat von ca. 80 Gew.-% aufwies (der Rest bestand im wesentlichen aus anorganischen Salzen und Wasser), wurde anschließend in einem 160 Liter-Lödige-Mischer auf 20 kg einer handelsüblichen Kieselsäure aufgebracht und die Mischung wurde erstarrt. Danach wurde die erstarrte Mischung auf eine Partikelgröße von 350 bis 1600 Mikrometer gesiebt. Das Grobgut größer als 1600 Mikrometer wurde gemahlen und anschließend wieder gesiebt. So wurde ein körniges Granulat mit einem Gehalt von ca. 60 Gew.-% an N-Methylmorpholinium-acetonitril-hydrogensulfat erhalten.

## Patentansprüche

1. Verfahren zur Herstellung von körnigen N-Alkylammoniumacetonitril-Salzen der allgemeinen Formel I
R²R³N⁺R¹-CR⁴R⁵-CN Y⁻ (I)
in der
R¹ eine C₁- bis C₂₄-Alkylgruppe, welche durch nicht benachbarte Sauerstoffatome unterbrochen sein oder zusätzlich Hydroxylgruppen tragen kann, eine C₄bis C₂₄-Cycloalkylgruppe, eine C₇- bis C₂₄-Alkarylgruppe oder eine Gruppierung der Formel -CR⁴R⁵-CN bedeutet,
R² und R³ jeweils unabhängig voneinander die Bedeutung von R¹ aufweisen oder zusammen einen gesättigten vierbis neungliedrigen Ring mit wenigstens einem C-Atom und wenigstens einem weiteren Heteroatom aus der Gruppe Sauerstoff, Schwefel und Stickstoff darstellen,
R⁴ und R⁵ jeweils unabhängig voneinander Wasserstoff, C₁- bis C₂₄-Alkylgruppen, welche durch nicht benachbarte Sauerstoffatome unterbrochen sein oder zusätzlich Hydroxylgruppen tragen können, C₄- bis C₂₄-Cycloalkylgruppen oder C₇- bis C₂₄-Alkarylgruppen bezeichnen und
Y⁻ für ein Sulfat- oder Hydrogensulfat-Anion in der entsprechenden stöchiometrischen Menge steht,
aus einer wäßrigen Lösung der Verbindung der allgemeinen Formel II
R²R³N⁺R¹-CR⁴R⁵-CN R⁶O-SO₂-O⁻ (II)
in der R¹ bis R⁵ die oben genannten Bedeutungen aufweisen und R⁶ für C₁- bis C₄-Alkyl steht, indem man diese wäßrige Lösung bei einer Temperatur von 80°C bis 250°C und einem Druck von 10 mbar bis 2 bar zu einer Schmelze eindampft, anschließend die Schmelze erstarren läßt, wobei während oder im Anschluß an das Eindampfen übliche Trägermaterialien und/oder Hilfsmittel zugegeben werden können, und die erhaltene erstarrte Verbindung I in die gewünschte körnige Form überführt, **dadurch gekennzeichnet, daß** die Hydrolyse des Gegenions R⁶O-SO₂-O⁻ zu Y⁻ thermisch, d.h. ohne Zugabe von Säure, erfolgt.

2. Verfahren zur Herstellung von körnigen N-Alkylammoniumacetonitril-Salzen I nach Anspruch 1, bei denen R¹ eine C₁- bis C₄-Alkylgruppe oder einen Benzylrest bedeutet und R⁴ und R⁵ Wasserstoff bezeichnen.

3. Verfahren zur Herstellung von körnigen N-Alkylammoniumacetonitril-Salzen I nach Anspruch 1 oder 2, bei denen R² und R³ zusammen einen gesättigten sechsgliedrigen Ring mit 5 C-Atomen oder mit 4 C-Atomen und einem Sauerstoff oder einem Stickstoffatom darstellen.

4. Verfahren zur Herstellung von körnigem N-Methylmorpholiniumacetonitril-hydrogensulfat aus einer wäßrigen Lösung von N-Methylmorpholiniumacetonitril-methylsulfat nach den Ansprüchen 1 bis 3.

5. Verfahren zur Herstellung von körnigen N-Alkylammoniumacetonitril-Salzen I nach den Ansprüchen 1 bis 4, **dadurch gekennzeichnet, daß** man die Eindampfung bei einer Temperatur von 100°C bis 160°C durchführt.

6. Verfahren zur Herstellung von körnigen N-Alkylammoniumacetonitril-Salzen I nach den Ansprüchen 1 bis 5, **dadurch gekennzeichnet, daß** man die Eindampfung bei einem Druck von 250 bis 900 mbar durchführt.

7. Verfahren zur Herstellung von körnigen N-Alkylammoniumacetonitril-Salzen I nach den Ansprüchen 1 bis 6, **dadurch gekennzeichnet, daß** man die Eindampfung kontinuierlich mit einer Verweilzeit von 5 bis 15 Minuten durchführt.

8. Verfahren zur Herstellung von körnigen N-Alkylammoniumacetonitril-Salzen I nach den Ansprüchen 1 bis 7, **dadurch gekennzeichnet, daß** man die Kondensationswärme der bei der Eindampfung entstehenden Brüden zur Vorwärmung der wäßrigen Lösung der Verbindung II benutzt.

9. Verfahren zur Herstellung von körnigen N-Alkylammoniumacetonitril-Salzen I nach den Ansprüchen 1 bis 8, **dadurch gekennzeichnet, daß** man die Eindampfung bis zu einem Restwassergehalt von maximal 30 Gew.-% vornimmt.

10. Verfahren zur Herstellung von körnigen N-Alkylammoniumacetonitril-Salzen I nach den Ansprüchen 1 bis 9, **dadurch gekennzeichnet, daß** man während oder im Anschluß an das Eindampfen ein oder mehrere Trägermaterialien aus der Gruppe Natriumsulfat; Kieselsäuren und Zeolithe zugibt.

## Claims

1. A process for the preparation of granular N-alkylammoniumacetonitrile salts of the formula I
R²R³N⁺R¹-CR⁴R⁵-CN Y⁻ (I)
in which
R¹ is a C₁- to C₂₄-alkyl group, which can be interrupted by nonadjacent oxygen atoms or can carry additional hydroxyl groups, a C₄- to C₂₄-cycloalkyl group, a C₇- to C₂₄-alkaryl group or a group of the formula -CR⁴R⁵-CN,
R² and R³ in each case independently of one another have the meaning of R¹ or together are a saturated 4- to 9-membered ring having at least one carbon atom and at least one other heteroatom from the group consisting of oxygen, sulfur and nitrogen,
R⁴ and R⁵ in each case independently of one another are hydrogen, C₁- to C₂₄-alkyl groups, which can be interrupted by nonadjacent oxygen atoms or can additionally carry hydroxyl groups, C₄- to C₂₄-cycloalkyl groups or C₇- to C₂₄-alkaryl groups, and
Y⁻ is a sulfate or hydrogensulfate anion in the corresponding stoichiometric amount,
from an aqueous solution of the compound of the formula II
R²R³N⁺R¹-CR⁴R⁵-CN R⁶O-SO₂-O⁻ (II)
in which R¹ to R⁵ are as defined above and R⁶ is C₁- to C₄-alkyl,
by evaporating this aqueous solution at a temperature of from 80°C to 250°C and a pressure of from 10 mbar to 2 bar to give a melt, then allowing the melt to solidify, where, during or following the evaporation, customary carrier materials and/or auxiliaries can be added, and the resulting solidified compound I is converted into the desired granular form, wherein the hydrolysis of the counterion R⁶O-SO₂-O- to Y-takes place thermally, i.e. without the addition of acid.

2. A process for the preparation of granular N-alkylammoniumacetonitrile salts I as in claim 1, in which R¹ is a C₁- to C₄-alkyl group or a benzyl radical, and R⁴ and R⁵ are hydrogen.

3. A process for the preparation of granular N-alkylammoniumacetonitrile salts I as in claim 1 or 2, in which R² and R³ together are a saturated six-membered ring having 5 carbon atoms or having 4 carbon atoms and one oxygen or one nitrogen atom.

4. A process for the preparation of granular N-methylmorpholiniumacetonitrile hydrogen sulfate from an aqueous solution of N-methylmorpholiniumacetonitrile methylsulfate as in claims 1 to 3.

5. A process for the preparation of granular N-alkylammoniumacetonitrile salts I as in claims 1 to 4, wherein the evaporation is carried out at a temperature of 100°C to 160°C.

6. A process for the preparation of granular N-alkylammoniumacetonitrile salts I as in claims 1 to 5, wherein the evaporation is carried out at a pressure of from 250 to 900 mbar.

7. A process for the preparation of granular N-alkylammoniumacetonitrile salts I as in claims 1 to 6, wherein the evaporation is carried out continuously with a residence time of from 5 to 15 minutes.

8. A process for the preparation of granular N-alkylammoniumacetonitrile salts I as in claims 1 to 7, wherein the heat of condensation of the vapors produced during evaporation is used to prewarm the aqueous solution of the compound II.

9. A process for the preparation of granular N-alkylammoniumacetonitrile salts I as in claims 1 to 8, wherein the evaporation is carried out until the residual water content is at most 30 % by weight.

10. A process for the preparation of granular N-alkylammoniumacetonitrile salts I as in claims 1 to 9, wherein, during or following the evaporation, one or more carrier materials from the group consisting of sodium sulfate, silicas and zeolites are added.

## Revendications

1. Procédé de préparation de sels de N-alkylammonium-acétonitrile, granulaires, de la formule générale I :
R²R³N⁺R¹-CR⁴R⁵-CN Y⁻ (I)
dans laquelle :
R¹ représente un radical alkyle en C₁ à C₂₄, qui peut être interrompu par des atomes d'oxygène non voisins ou peut porter des radicaux hydroxyle supplémentaires, un radical cycloalkyle en C₄ à C₂₄, un radical alkaryle en C₇ à C₂₄, ou un groupement de la formule -CR⁴R⁵-CN ;
R² et R³ présentent chaque fois indépendamment l'un de l'autre, la signification de R¹ ou représentent ensemble, un cycle saturé ayant 4 à 9 membres, avec au moins un atome C et au moins un hétéroatome du groupe de l'oxygène, du soufre et de l'azote ;
R⁴ et R⁵ représentent chaque fois indépendamment l'un de l'autre, l'hydrogène, un radical alkyle en C₁ à C₂₄, qui peut être interrompu par des atomes d'oxygène non voisins ou peut porter des radicaux hydroxyle supplémentaires, un radical cycloalkyle en C₄ à C₂₄, un radical alkaryle en C₇ à C₂₄, et
Y⁻ représente un anion sulfate ou hydrogénosulfate en la quantité stoechiométrique appropriée,
à partir d'une solution aqueuse du composé de la formule générale II :
R²R³N⁺R¹-CR⁴R⁵-CN R⁶O-SO₂-O⁻ (II)
dans laquelle R¹ à R⁵ présentent les significations indiquées ci-dessus et R⁶ représente alkyle en C₁ à C₄, en ce que l'on évapore cette solution aqueuse à une température allant de 80°C à 250°C et sous une pression de 10 mbar à 2 bar en une masse fondue, ensuite la masse fondue peut prendre en masse, où pendant ou à la suite de l'évaporation, on peut ajouter les matériaux support et/ou auxiliaires usuels et le composé I, pris en masse, obtenu peut être converti en une forme granulaire souhaitée, **caractérisé en ce que** l'hydrolyse du contre-ion R⁶O-SO₂-O⁻ en Y⁻ est réalisée thermiquement, à savoir sans addition d'acide.

2. Procédé de préparation de sels de N-alkylammonium-acétonitrile, granulaires I selon la revendication 1, dans lequel R¹ représente un radical alkyle en C₁ à C₄ ou un reste benzyle et R⁴ et R⁵ représentent hydrogène.

3. Procédé de préparation de sels de N-alkylammonium-acétonitrile, granulaires I selon la revendication 1 ou 2, dans lequel R² et R³ représentent ensemble, un cycle saturé à 6 membres, avec 5 atomes C ou avec 4 atomes C et un oxygène ou un azote.

4. Procédé de préparation d'hydrogénosulfate de N-méthyl-morpholiniumacétonitrile, granulaire à partir d'une solution aqueuse de méthylsulfate de N-méthyl-morpholiniumacétonitrile selon les revendications 1 à 3.

5. Procédé de préparation de sels de N-alkylammonium-acétonitrile, granulaires I selon les revendication 1 à 4, **caractérisé en ce que** l'on réalise l'évaporation à une température allant de 100°C à 160°C.

6. Procédé de préparation de sels de N-alkylammonium-acétonitrile, granulaires I selon les revendication 1 à 5, **caractérisé en ce que** l'on réalise l'évaporation à une pression allant de 250 à 900 mbar.

7. Procédé de préparation de sels de N-alkylammonium-acétonitrile, granulaires I selon les revendication 1 à 6, **caractérisé en ce que** l'on réalise l'évaporation de manière continue avec un temps de séjour de 5 à 15 minutes.

8. Procédé de préparation de sels de N-alkylammonium-acétonitrile, granulaires I selon les revendication 1 à 7, **caractérisé en ce que** l'on utilise la chaleur de condensation des vapeurs chaudes formées lors de l'évaporation pour le préchauffage de la solution aqueuse du composé II.

9. Procédé de préparation de sels de N-alkylammonium-acétonitrile, granulaires I selon les revendication 1 à 8, **caractérisé en ce que** l'on réalise l'évaporation jusqu'à une teneur résiduelle en eau de maximum 30% en poids.

10. Procédé de préparation de sels de N-alkylammonium-acétonitrile, granulaires I selon les revendication 1 à 9, **caractérisé en ce que**, pendant ou à la suite de l'évaporation, on ajoute un ou plusieurs matériaux support du groupe du sulfate d'ammonium, de l'acide silicique et des zéolithes.
